# EUROPEAN PATENT SPECIFICATION

(11) **EP 4 153 052 B1**
(45) Date of publication and mention of the grant of the patent: **12.08.2026**
(21) Application number: 21729244.0
(22) Date of filing: 21.05.2021
(51) Int. Cl.: A61B 5/273, A61B 5/291

(54) **EXPANDABLE ELECTRODE SET**
EXPANDIERBARER ELEKTRODENSATZ
ENSEMBLE D'ÉLECTRODES EXTENSIBLE

(30) Priority: 21.05.2020 US 202016880104; 21.07.2020 TW 109124603
(43) Date of publication of application: 29.03.2023
(73) Proprietor: Bioserenity Medical Devices Group, 75013 Paris (FR)
(72) Inventor: ARFAOUI, Nadia, 78400 Chatou (FR); ARISTIDES, Alexandre, 75013 Paris (FR); FROUIN, Marc, 75013 Paris (FR); GUERMONPREZ, Philippe, 75013 Paris (FR); KOCA, Ekin, 75013 Paris (FR); MERCHET, Maylis, 75013 Paris (FR); PARIZEL, Fabian, 75013 Paris (FR); PROT, Pierre, 75013 Paris (FR)
(74) Representative: Icosa
(86) International application number: PCT/EP2021/063681
(87) International publication number: WO 2021/234156

(56) References cited:
- EP-B1- 2 654 556
- AU-A- 6 558 101
- US-A1- 2017 019 988
- US-A1- 2019 313 930
- US-B1- 8 019 402

## Description

### FIELD OF THE DISCLOSURE

The present disclosure pertains to the field of the systems and methods for acquiring a physiological signal from a subject. In particular, the disclosure relates to a set of electrodes for measuring electrical physiological signal.

### BACKGROUND OF THE DISCLOSURE

Acquiring electrophysiological signals is of great importance in current medical technologies. Electrical activity of heart (electrocardiogram ECG), brain (electroencephalogram EEG), nerves (electromyogram EMG) or pregnant women (fetus electrocardiogram or fECG) or other electrical measurement (electrooculography (EOG) for eyes, ERG EEG for intestine activities, and the like) are commonly recorded for diagnostic or monitoring purpose. In addition, stimulation by electric signals or imaging based on impedance measurements of a part of the body of a subject (EIT: electrical impedance tomography) are spreading quickly in medical practice. Achieving correct measurements of electrophysiological signals requires to locate precisely measuring electrodes. Correct placement of electrodes for stimulation or imaging is necessary for accurate readings.

Similarly, when a given pathology required co deployment of electrodes (or other sensors) with specific placement and other type of skin contact sensors like light emitting diodes and photoreceptors or sensors created using Silicon integrated cells (MEMS) it is required for correct measurement to reach correct positions precisions in many medical cases.

### SUMMARY

This disclosure thus relates to an electrode set, comprising:
- a first node and a second node, the first node comprising a first pad for receiving electromagnetic energy from a first portion of a part being studied, the second node comprising a second pad;
- a connector connecting the first node to the second node, the connector formed from a shape that provides for a consistent deformation from a first distance between the first node and the second node to one or more second distances between the first node and the second node as a pull force is applied to the first node or the second node, wherein in an undeformed state the first node and the second node are planar and in a deformed state the first node and the second node are non-planar, wherein a length of the connector remains constant from the undeformed state to the deformed state;
- at least one alignment marker affixed to either the first node or the second node, the at least one alignment marker to be removably affixed to a landmark on the subject of the part being studied, wherein the at least one alignment marker is configured to transfer the pull force to either the first node or the second node as the alignment marker is being affixed to the landmark.
The wording of connector formed from a shape that provides for a consistent deformation, may also be understood as connector having a shape configured to provide a consistent deformation. Advantageously, the present electrode set when it is in its undeformed state is planar which allow for compact storage of a large number of electrode sets. This aspect is particularly interesting for example for ambulances where a large number of different medical system/devices has to be stored for moving around.

According to one embodiment, the second pad for receiving electromagnetic energy from a second portion of the part being studied or for sending a signal into the first portion of a part being studied

According to one embodiment, the electrode set further comprises a first wire within the connector, the first wire in electrical communication with the first pad of the first node and extending to a measurement lead.

According to one embodiment, the electrode set further comprises a second wire within the connector, the second wire having in electrical communication with the second pad of the second node and extending to the measurement lead, wherein the measurement lead terminates at a measurement connector to be inserted into a monitoring/input device.

According to one embodiment, the electrode set further comprises a plurality of third nodes, at least a portion of the plurality of third nodes comprising a pad for receiving electromagnetic energy from a plurality of third portions of the part being studied; and a plurality of second connectors connecting at least one of the plurality of third nodes to either the first node or the second node, the plurality of second connectors formed from the shape that provides for consistent deformation from a first distance between at least a portion of the plurality of third nodes to one or more second distances between at least a portion of the plurality of third nodes as a pull force is applied to the at least one alignment marker, wherein in an undeformed state the plurality of third nodes are planar and in a deformed state the plurality of third nodes are non-planar.

According to one embodiment, the electrode set further comprises a plurality of second alignment markers affixed to one or more of the plurality of third nodes, the plurality of second alignment markers to be removably affixed to a plurality of second landmarks on the subject of the part being studied, wherein the plurality of second alignment markers are configured to transfer the pull force to at least a portion of the plurality of third nodes as the plurality of second alignment markers are being affixed to the landmark.

According to one embodiment, the electrode set further comprises a pad stiffener affixed to the first node, wherein the pad stiffener secures an electrical connection between the pad and the first wire.

According to one embodiment, the connector comprises a plastic substrate, polyimide, polyethylene, polyether ether ketone (PEEK), or a non-conductive polyester or polymer.

According to one embodiment, the shape comprises a sinusoidal shape. This embodiment, advantageously allows the spacing between the nodes to be changed from a first distance to one or more second distances depending on how much the connectors are pulled.

According to one embodiment, the shape comprises a spiral, a double spiral, a horseshoe, or an angular shape.

According to one embodiment, the first pad and/or the second pad comprises conductive or semi-conductive materials.

According to one embodiment, the first pad and/or the second pad comprises copper, aluminum, stainless steel, gold, silver, or alloys thereof.

According to one embodiment, the first wire and/or the second wire comprises copper, aluminum, stainless steel, gold, silver, or alloys thereof.

According to one embodiment, the first pad comprises a first passing hole and the second pad comprises a second passing hole, the first passing hole and the second passing hole being openings that allow for an injection of an electroconductive material enhancing electrical contact with skin.

According to one embodiment, the electrode set further comprises a third node, wherein the third node comprises a third pad to transmit electromagnetic energy.

According to one embodiment, the electromagnetic energy is near infrared light, infrared light, or visible light.

According to one embodiment, the electrode set further comprises a fourth node, wherein the fourth node comprises a fourth pad to receive electromagnetic energy resulting from the electromagnetic transmission from the third pad.

According to one embodiment, the electrode set further comprises a third node for receiving a sensor.

The present disclosure also relates to a method of measuring signals of a part of a subject, the method comprising:
- placing an electrode set proximate to the part to be measured, the electrode set comprising:
- a first node and a second node, the first node comprising a first pad for receiving electromagnetic energy from a first portion of a part being studied, the second node comprising a second pad for receiving electromagnetic energy from a second portion of the part being studied;
- a connector connecting the first node to the second node, the connector formed from a shape that provides for a consistent deformation from a first distance between the first node and the second node to one or more second distances between the first node and the second node as a pull force is applied to the first node or the second node, wherein in an undeformed state the first node and the second node are planar and in a deformed state the first node and the second node are non-planar, wherein a length of the connector remains constant from the undeformed state to the deformed state;
- at least one alignment marker affixed to either the first node or the second node, the at least one alignment marker to be removably affixed to a landmark on the subject of the part being studied, wherein the at least one alignment marker is configured to transfer the pull force to either the first node or the second node as the alignment marker is being affixed to the landmark;
- a first wire within the connector, the first wire in electrical communication with the first pad of the first node and extending to a measurement lead; and
- a second wire within the connector, the second wire having in electrical communication with the second pad of the second node and extending to the measurement lead, wherein the measurement lead terminates at a measurement connector to be inserted into a monitoring/input device:
   - affixing the at least one alignment marker to the landmark;
   - connecting the measurement lead to a monitoring/input device; and
   - instantiating a monitoring application in the monitoring/input device to commence measuring the electrophysiological signals of the part of the subject.

According to one embodiment, the method further comprises injecting an electroconductive, sonic, or light transmitting material through a first passing hole of the first pad and a second passing hold of the second pad.

According to one embodiment, the connector comprises a plastic substrate, polyimide, polyethylene, polyether ether ketone (PEEK), or a non-conductive polyester or polymer.

According to one embodiment, the shape comprises a sinusoidal shape.

According to one embodiment, the shape comprises a spiral, a double spiral, a horseshoe, or an angular shape.

According to one embodiment, the first pad and/or the second pad comprises conductive or semi-conductive materials.

According to one embodiment, the first pad and/or the second pad comprises copper, aluminum, stainless steel, gold, silver, or alloys thereof.

The present disclosure relates as well to a sensor set system comprising:
- a sensor set (100) according to any one of the embodiments cited above, wherein the monitoring/input device is configured to receive data of electrophysiological signals received from the first pad or the second pad; and
- a monitoring service in communication with the monitoring/input device for receiving the data of the electrophysiological signals or transmitting instructions to the monitoring/input device.

### BRIEF DESCRIPTION OF THE DRAWINGS

The detailed description is set forth with reference to the accompanying figures. In the figures, the left-most digit(s) of a reference number identifies the figure in which the reference number first appears. The use of the same reference numbers in different figures indicates similar or identical items or features.
FIG. 1 depicts an expandable electrode set in a non-deployed state, in accordance with some examples of the present disclosure.
FIG. 2 depicts an electrode set in a deployed state, in accordance with some examples of the present disclosure
FIG. 3 illustrates electrical wiring in an expandable electrode set, in accordance with some examples of the present disclosure.
FIG. 4 is a close-up view of an example node used in an expandable electrode set, in accordance with some examples of the present disclosure.
FIG. 5 is a schematic diagram depicting an electrode set system, in accordance with some examples of the present disclosure.
FIG. 6 is a flowchart depicting a method of using an expandable electrode set, in accordance with some examples of the present disclosure.
FIG. 7 is a flowchart depicting a method of manufacturing an expandable electrode set, in accordance with some examples of the present disclosure.
FIG. 8 is a depicts a component level view of a monitoring/input device for use with the systems and methods described herein, in accordance with some examples of the present disclosure.

### DETAILED DESCRIPTION

Examples of the present disclosure can comprise systems and methods for providing and using an expandable sensor set. When using a conventional sensor set to measure three-dimensional body parts, it is often difficult to properly align the sensors at the correct position on the body (part) of a patient to try to get as accurate of a reading as possible. This often limits the use of conventional sensor sets to hospitals or other facilities in which a technician is available for the installation. Because of the need for specialists to install conventional sensor sets, the costs to use conventional sensor sets can be prohibitively expensive as well as inconvenient, this in turns create limited capacities to leverage the data that can be acquired in many medical cases. It should be noted that, while some of the figures are described in terms of installation onto a subject by a second person, the presently disclosed subject matter is not limited in that manner, as various examples of the presently disclosed subject matter may be installed by the subject themselves.

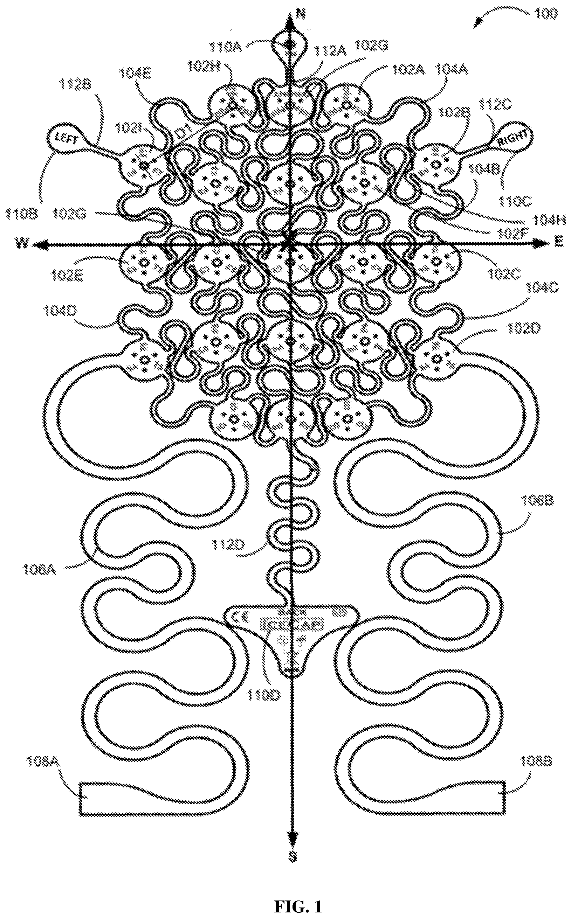

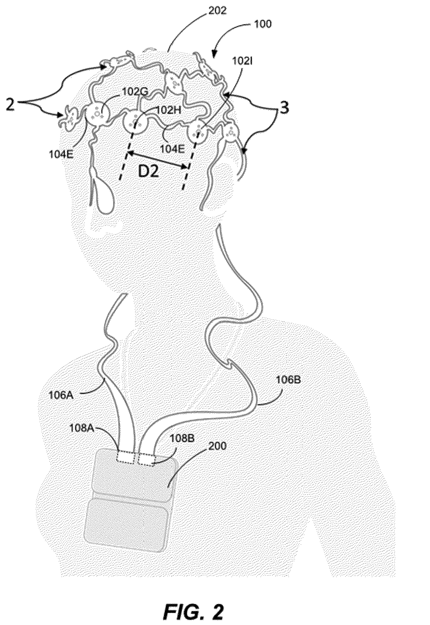

FIG. 1 depicts a top-down view of an expandable electrode set 100 in a non-deployed state. It should be noted that although some of the description herein is described in terms of an "electrode" or an "electrode set," the presently disclosed subject matter is not limited to electrodes, as the description using electrodes is merely exemplary and illustrative. As used herein, "non-deployed or undeployed" means that the electrode set 100 is not installed on a body part and "deployed" means that the electrode set 100 is partially or fully installed on a body part. Referring to FIG. 2, the electrode set 100 is shown in a deployed state. In the non-deployed state illustrated in FIG. 1, the electrode set 100 is substantially flat, meaning that when placed on a flat surface, all or substantially all of a bottom surface of the electrode set 100 proximate to the flat surface will be in contact with the surface. In the deployed state illustrated in FIG. 2, the electrode set 100 is partially deformed to wrap around a 3D body part of a patient or subject to be studied. In some examples, the deformation may be termed "warping," wherein both terms are interchangeable. For example, nodes 102H and 102I, connected by connector 104E, are shown in FIG. 1 to have a distance of D1 between the nodes 102I and 102H, whereas in FIG. 2, the distance between the nodes 102I and 102H is illustrated as D2, which is greater than D1.

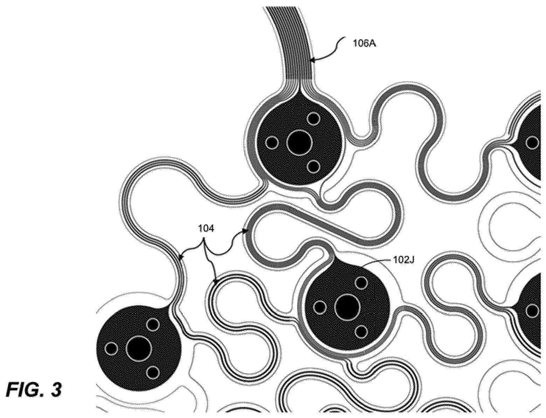

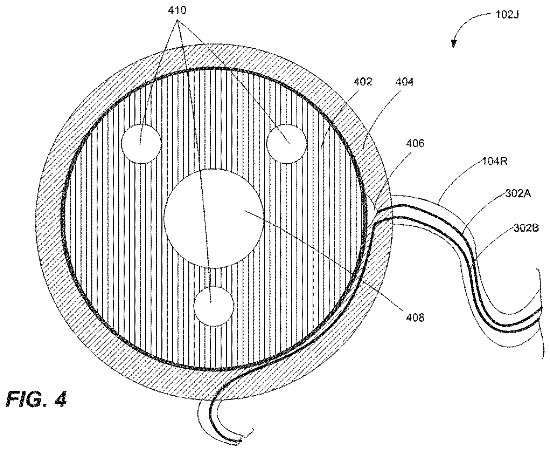

Referring back to FIG. 1, the electrode set 100 includes nodes 102A-102F (collectively referred to herein as the "nodes 102," and individually the "node 102A," the "node 102B," and so forth) and connectors 104A-104E (collectively referred to herein as the "connectors 104," and individually the "connector 104A," the "connector 104B," and so forth). It is noted that FIG. 1 includes additional nodes and connectors not labeled, which is merely for purposes of illustration. The internal construction of the nodes 102 and the connectors 104 are described in more detail in FIGS. 3 and 4. In use, the nodes 102 are used to sense (measure or detect) electrophysiological signals that are the result of electrical activity of a particular body part. It is noted that the shapes of the various components of the electrode set 100 illustrated in FIG. 1 are merely exemplary and may have different shapes depending on a particular use. For example, the nodes 102 may be circular as illustrated in FIG. 1, but may also be ellipses, egg-shaped, squares, rectangles, and/or polygons, or combinations thereof. In some examples, the nodes 102 may be used to impart a current into the subject being tested to measure impedance. These and other uses of the nodes 102 as electro- or electromagnetic devices are considered to be within the scope of the presently disclosed subject matter.

The electrode set 100 further includes measurement leads 106A and 106B and measurement connectors 108A and 108B. The measurement leads 106A and 106B receive electrical signals from the nodes 102 through the connectors 104. The measurement leads 106A and 106B have within them wires from each of the nodes 102 that run from the nodes 102 to the measurement leads 106A and 106B. The measurement leads 106A and 106B are connected to a device that measures the electrical signals from the nodes 102 (shown in more detail in FIG. 2).

As noted above, in conventional electrode sets, a technician or other qualified individual is often required in order to ensure that the electrode set is properly positioned on a body part. The reason for this is that the nodes that measure bodily electrical activity need to be placed at certain points on the body to get as accurate of a reading as possible. The electrode set 100 of FIG. 1 provides various mechanisms that allow various users, including untrained people, to properly place the electrode set 100 on a body party (e.g. a head or a belly of a pregnant woman).

A first mechanism that allows for the proper placement of the electrode set 100 on a body part are alignment markers 110A-110D (collectively referred to herein as the "alignment markers 110," and individually the "alignment marker 110A," the "alignment marker 110B," and so forth). The alignment markers 110 are used by a person installing the electrode set 100 to properly align the electrode set 100. The alignment markers 110 are configured to be put in contact with a predefined anatomical landmark on a person to be studied. The landmarks can be based on various factors, including standards defined by a medical community to place the nodes 102 so as to record correctly electrophysiological signals. Such systems exist for ECG, EEG, EMG, and/or fECG or other electrical physiological signal as well as placement of other sensors sources for other measurement technologies. However, the presently disclosed subject matter does not require the use of specific landmarks, as other locations on a body may be used and are considered to be within the scope of the presently disclosed subject matter. For example, the ears, nose, belly button, or other landmark may be used for the proper placement of the electrode set 100. It should also be noted that the electrode set 100 is not limited to use on humans, as the electrode set 100 may be used on non-human subjects. In the example illustrated in FIG. 1, the alignment markers 110 are located according to nasion, inion and both tragi anatomical landmarks in the international 10-20 system or variants thereof, though as mentioned herein, other landmarks may be used and are considered to be within the scope of the presently disclosed subject matter.

A person installing the electrode set 100 places and temporarily affixes (using tape or other adhesive appropriate for use on a body) the one or more alignment markers 110 on one or more locations (i.e. the anatomical landmarks). In the example illustrated in FIG. 1, there are four alignment markers 110, although as previously mentioned, there may be more than four or fewer than four depending on the particular configuration of the electrode set 100. When placing one of the alignment markers 110 on the anatomical landmark, the placement of the alignment marker 110 advantageously exerts a force on the node 102 closest to the alignment marker 110 through marker connectors 112A-112D (collectively referred to herein as the "marker connectors 112," and individually the "marker connector 112A," the "marker connector 112B," and so forth). In one advantageous example, the placement of the alignment marker 110C exerts a pulling force on the node 102B through the marker connector 112C in a direction generally in line with force vector XE. Similarly, the placement of the alignment marker 110A exerts a force generally in line with force vector XN, the placement of the alignment marker 110B exerts a force generally in line with force vector XW, and the placement of the alignment marker 110D exerts a force generally in line with force vector XS.

The action of the pulling of the electrode set 100 in the direction of two or more force vectors (such as XE, XN, XW, and XS) causes the electrode set 100 to warp or deform. As used herein, "warp" refers to a material that has been deformed from a planar state (as illustrated in FIG. 1) to a three-dimensional state (as illustrated by way of example in FIG. 2). The electrode set 100 is designed with materials that provide an appropriate stretch force that counters the pulling force to correctly align the nodes 102 onto the one or more anatomical landmarks. For example, an elastic force (i.e. the force that occurs when a deformed object tries to return to its original shape) that is too low may cause various nodes to be too easily pulled in a particular direction. In this example, the rigidity of the structure of the electrode set 100 and its nodes 102 and connectors 104 is insufficient to provide a controlled and specific deployment of the nodes 102 of the electrode set 100. In another example, if the rigidity of the structure of the electrode set 100 is too great, meaning the elastic force is relatively significant, the structure of the electrode set 100 may require heavy glues or adhesives to keep the alignment markers 110 in place and may place an undue strain on the material of the electrode set 100, among other disadvantages.

Thus, construction of the electrode set 100 and its components, especially the connectors 104, are designed to provide a balance between rigidity and flexibility. In the example illustrated in FIG. 1, a sinusoidal shape constructed with particular materials advantageously achieves this balance. It should be understood that the shapes and materials are examples, as other shapes and materials may be used. For example, other shapes such as spiral-shaped, double spiral-shaped, horseshoe-shaped or angular-shaped may be used. The shape of the connectors 104 of the electrode set is designed to allow for a planar configuration when not deployed while allowing for a non-planar configuration during use.

Advantageously, the sinusoidal shape also allows the spacing between the nodes 102 to be changed from a first distance to one or more second distances depending on how much the connectors 104 are pulled. The one or more second distances may be used to allow the electrode set 100 to be deployed in various uses. **In** one embodiment, the ratio of a first distance between two nodes linked by a connector, such as the nodes 102B and 102F connected by the connector 104H, in the deployed configuration and the distance between the same two nodes linked by the same connector element in the undeployed configuration is greater than 1.05, and in some examples, greater than the ratios of 1.05 and up to 2.0, though greater ratios may be achievable depending on the particular materials, dimensions, and the like.

Further, the sinusoidal shape along with the predetermined elastic force provided by the connectors 104 advantageously allow for the electrode set 100 to be used on various sizes and shapes of body parts. For example, the electrode set 100 may be used for skulls or abdomens, as well as differently shaped body parts, including those of various cultures and ethnicities. As the electrode set 100 is installed on a body part, the sinusoidal shape and the elastic force cause the electrode set 100 to deform in a predetermined manner. For example, as the electrode set 100 is deformed to fit over a body part, the elastic force and the sinusoidal shape cause the nodes 102 of the electrode set 100 to space apart at distances in the direction of the force vectors that allow for a proper placement of the nodes 102 on the locations of the body part to be measured. This means that when installed, the electrode set 100 will not have an area of nodes 102 that remain bunched together at or near pre-deformation distances and other nodes 102 that are spread too far apart at or near post-deformation distances. The consistent deformation across all force vectors allows the electrode set 100 to be used on various body sizes and shapes. During the process from the undeformed to the deformed state, the length of the connectors 104 remain the same, meaning the connectors 104 don't stretch, but rather, their shape changes from a sinusoidal to linear shape.

In some examples, the connectors 104 are constructed using a polyimide, polyethylene, polyether ether ketone (PEEK), or other fully or partially insulative polymer. In some examples, the connectors 104 (including any internal components such as copper tracks or wiring) preferably have a thickness within a range of 90 µm to 200 µm, a range of 100 µm to 170 µm, and in a more preferable configuration, a range of thickness from 118 µm to 122 µm. In some examples, the thickness of the connectors 104 is 120 µm with a tolerance of twenty percent (20%). It should be noted that the thickness of the connectors 104 may vary depending on the particular material used in order to provide a similar elastic force.

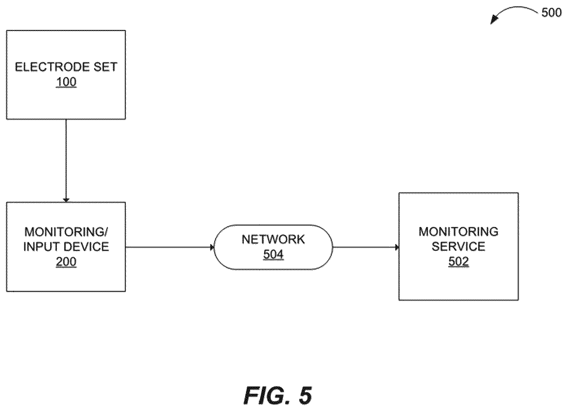

Referring back to FIG. 2, also illustrated is a monitoring/input device 200. In some examples, the monitoring/input device 200 provides the electrical power to allow the nodes 102 to be used to detect electrophysiological signals produced by the part 202 of the human being studied, e.g. the head illustrated by way of example in FIG. 2. In the example in which the nodes 102 are being used to image the part 202, the monitoring/input device 200 provides the electrical power through the measurement leads 106A and 106B to allow for the imaging. As illustrated, the measurement leads 106A and 106B are connected to the monitoring/input device 200 by the insertion of the measurement connectors 108A and 108B into appropriate ports (not shown) of the monitoring/input device 202. The monitoring/input device 200 may record data for later transfer to a system for diagnosis/measurement and/or may have internal communication capabilities that allow the monitoring/input device 200 to transmit the data for use (explained in more detail in FIG. 5).

FIG. 3 illustrates electrical wiring in the electrode set 100, in accordance with some examples of the present disclosure. Shown in FIG. 3 are the measurement lead 106A and nodes 104. Within the nodes 104 and the measurement lead 106A (measurement lead 106B is similarly constructed) are wires that put the nodes 102 (for example, the node 102J in FIG. 3) in electrical communication with the measurement lead 106A, illustrated in more detail in FIG. 4.

FIG. 4 is a close-up view of the node 102J used in the electrode set 100, in accordance with some examples of the present disclosure. The node illustrated in FIG. 4 includes a pad 402, a pad stabilizer 404, and a pad stiffener 406. The pad 402 can be constructed of various conductive and semi-conductive materials including, but not limited to, copper, aluminum, stainless steel, and the like. An active area of the pad 402 (i.e. the area placed in contact or proximate to the surface of the part of the subject being studied) can comprise silver, silver chloride, electrically conductive silicone, electrically conductive polymer, or a plastic loaded with a conductive material such as carbon. The pad 402 is illustrated as being circular in shape, but other shapes, such as, but not limited to, a spiral, a double spiral, a horseshoe, or an angular shape, may be used and are considered to be within the scope of the presently disclosed subject matter. The pad 402 is stabilized and attached to the connector 104R by the pad stabilizer 404. The pad stabilizer envelops at least a portion of the pad 402, though the surface of the pad 402 that is designed to be placed in contact with the skin or other surface to be measured or detected has preferably little to no material. In another example, one or more of the pads, and the pads themselves, may be constructed of magnetic conductors (such as carbon) that may be advantageously used in applications such as magnetic resonance imaging.

The pad stiffener 406 is used to stiffen or secure the electrical connection between the pad 402 and a wire 302A. A wire 302B is used by another node 102. The wire 302A is use by a measurement device to detect electrical activity the subject being studied (the passive configuration) or, in an alternative configuration, deliver electrical energy (the active configuration). For example, in a passive configuration, the node 402 may be used to detect electrical activity from a subject being studied. In the active configuration, the node 402 may receive enough electrical energy from the wire 302A to allow for the imaging of a portion of the body by stimulating the subject with electrical signals. For example, a body part may be imaged by deploying the node 102J, applying a current through the wire 302A into the pad 402, recording potentials, and reconstructing an image from the potentials of the node 102J and other nodes 102.

The pad 402 of the node 102J further includes a passing hole 408. The passing hole 408 is an opening through the pad 402 and is advantageously used to allow an injection through the passing hole 408 for introducing a layer of an electroconductive material between the active area of the pad 402 and a portion of skin of the subject being studied while the pad 402 is proximate to the skin of the subject being studied. A type of electroconductive material may be gel used with EEG or ECG cup electrodes, though other types of electroconductive materials may be used and are considered to be within the scope of the presently disclosed subject matter. This embodiment is particularly advantageous since it allows to introduce the electroconductive gel between the pad and the subject skin which improves the signal quality. Notably when the portion to be studied is the head, the electroconductive gel allow to create a contact between the pad and the subject skin, through the hair of the subject. The pad 402 of the node 102J further includes orifices 410. The orifices 410 may be used to provide a means for affixing the node 102J in a manner similar to the passing hole 408 or may be used to allow air to escape when the node 102J is affixed, among other uses. The passing hole 408 may also be used to determine if sufficient gel or cream is dispensed, as the cream or gel may leak through the passing hole 408 when a sufficient amount is used. It should be noted that the node 102J may include more or fewer orifices 410 and more passing holes 408 or no passing holes 408. In alternative designs, this passing hole 408 is not present, and the gel may be dispensed under the electrode by lifting it to inject the gel. According to one embodiment, the user can choose which nodes to use as electrodes by applying only to the pads 402 of those nodes the electroconductive gel. The pads without electroconductive gel, which for example are in contact with the hair of the subject, will collect low or no signal that may be further discarded by software operations.

FIG. 5 is a schematic diagram depicting an electrode set system 500, in accordance with some examples of the present disclosure. In various examples, the electrode set 100 may be used to monitor or measure a part 202 of a body of a human. The electrode set 100 is in electrical communication with the monitoring/input device 200. As illustrated in FIG. 2, the electrode set 100 is connected to the monitoring/input device 200 by inserting the measurement leads 106A and 106B into the monitoring/input device 200. It is noted that the presently disclosed subject matter is not limited to removable measurement leads, as some configurations may include preinstalled measurement leads. These and other configurations are considered to be within the scope of the presently disclosed subject matter.

The electrode set system 500 further includes a monitoring service 502 communicatively connected to the monitoring/input device 200 through a network 504. The network 504 may be any type of network that communicatively connects the monitoring/input device 200 to the monitoring service 502, including, but not limited to, a Wi-Fi network, a local area network, or a cellular network. One of skill in the art will recognize that the systems and methods described herein can also be used with a variety of networks.

During use, the user of the electrode set 100 installs the electrode set on a body to be monitored and/or measured. The electrode set 100 is connected to the monitoring/input device 200. The monitoring/input device 200 is connected to the monitoring service 502. In some examples, the monitoring/input device 200 stores data locally while in use. In further examples, the monitoring/input device 200 transmits data to the monitoring service 502 while the electrode set 100 is in use or at anytime thereafter. In still further examples, the monitoring service 502 transmits instructions to the monitoring/input device 200 to configure the operation of the monitoring/input device 200. For example, while the monitoring/input device 200 is detecting bodily signals, the monitoring service 502 may detect an anomaly. The monitoring service 502 may send an instruction to the monitoring/input device 200 to modify its configuration from a measuring or detecting mode to an imaging mode to try to determine more information about the anomaly.

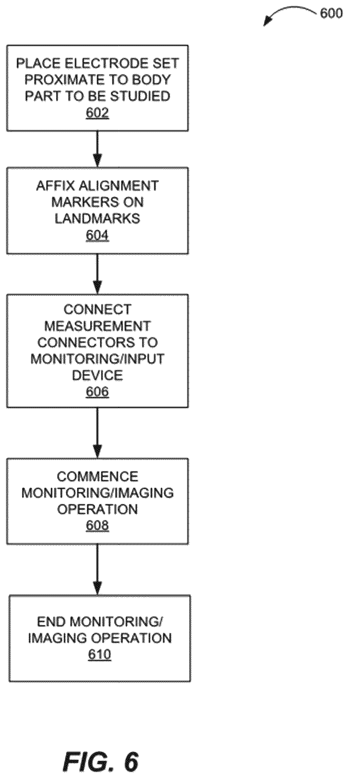

FIG. 6 is a flowchart depicting a process 600 of using an expandable electrode set, in accordance with some examples of the present disclosure. The process 600 and other processes described herein are illustrated as example flow graphs, each operation of which may represent a sequence of operations that can be implemented in hardware, software, or a combination thereof. In the context of software, the operations represent computer-executable instructions stored on one or more computer-readable storage media that, when executed by one or more processors, perform the recited operations. Generally, computer-executable instructions include routines, programs, objects, components, data structures, and the like that perform particular functions or implement particular abstract data types. The order in which the operations are described is not intended to be construed as a limitation, and any number of the described operations can be combined in any order and/or in parallel to implement the processes.

Referring to FIG. 6, the process 600 commences operation 602, where the electrode set is placed proximate to a part 202 of a human/anima/object to be studied. It should be understood that various aspects of the presently disclosed subject matter are described in terms of a human subject, though it should be understood that the presently disclosed subject matter is not limited to use on a human subject.

The process 600 continues to operation 604, where alignment markers 110 are affixed to landmarks on the part 202 or another location on the subject being studied. There may be one or more alignment markers 110 depending on the particular configuration of the electrode set 100. As the alignment markers 110 are being placed on the particular landmarks, the connectors 104 are pulled to cause a warping of at least a portion of the electrode set 100. This warping allows the electrode set 100 to transform from an undeployed, planar (or flat) configuration, to a deployed, three-dimensional confirmation that conforms to the general shape of the part 202 being monitored/imaged.

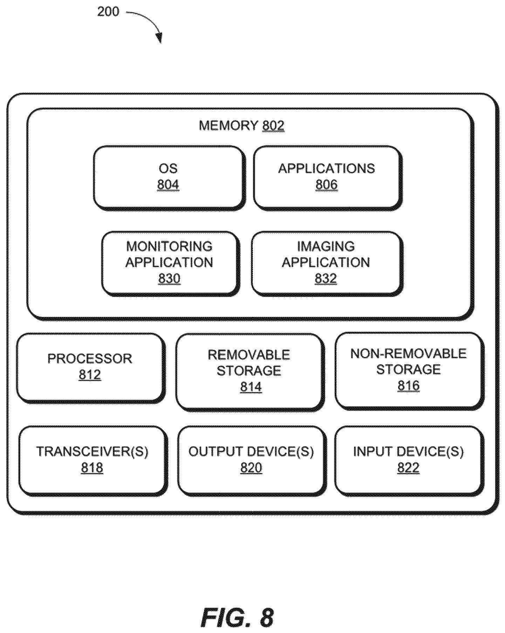

The process 600 continues to operation 606, where the measurement connectors 108 are connected to the monitoring/input device 200. In some examples, the monitoring/input device 200 may be executing a monitoring application or an imaging application (described in more detail in FIG. 8, below).

The process 600 continues to operation 608, where the monitoring or imaging of the part 202 is commenced. The process 600 thereafter ends at operation 610.

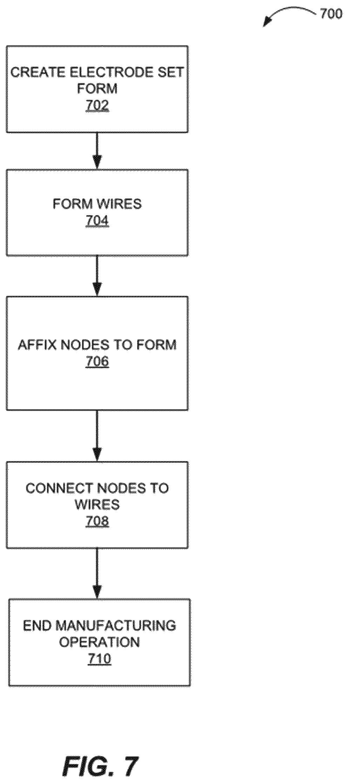

FIG. 7 is a flowchart depicting a process 700 for manufacturing the electrode set 100, in accordance with some examples of the present disclosure.

The process 700 commences at operation 702, where the electrode set 100 form is created. The form may be made using various processes using various materials. The form is the shape of the electrode set 100 structure. For example, in FIG. 1, the form includes the shape associated with the connectors 104, the alignment markers 110, and the like. In one example, the form is cut from a planar layer of a base material such as polyimide. The base layer can be an insulative or partially conductive layer of material upon which other materials can be placed. In some examples, the form comprises a single sheet of material, and in other examples, the form is constructed from two or more separate pieces of material. In some examples, the form may be multiple layers of material. It should be noted that instead of operation 702 being performed in the beginning, operation 702 may be performed after or before various other operations of process 700.

The process 700 continues at operation 704, where wires 302 are plated onto the form (or the base material if performed before operation 702). The wires 302 connect individual nodes 102 to the monitoring/input device 200 through the measurement leads 106A and 106B. The wires 302 may be formed using various plating or deposition technologies. The wires 302 may be formed from various conductive or semiconductive materials such as, but not limited to, copper, aluminum, gold, silver, and alloys thereof. The thickness of the wires 302 may vary, but in some examples, are between 0.3 and 0.5 µm.

The process 700 continues to operation 706, where the nodes 104 are affixed to the form. The nodes may be preformed metal discs of various conductive or semiconductive materials such as, but not limited to, copper, aluminum, gold, silver, and alloys thereof.

The process 700 continues to operation 708, where the nodes 104 are affixed to the wires 302 thru the pad stiffener 406 for each of the nodes 102. The pad stiffeners 406 may be formed from various materials, including polyimide or other polymers that provide sufficient structure support for the connection between the wires 302 and the pads 402.

The process 700 ends at operation 710.

FIG. 8 monitoring/input device for use with the systems and methods described herein, in accordance with some examples of the present disclosure. FIG. 8 illustrates the monitoring/input device 200 of FIG. 2 and FIG. 5, by way of example. The monitoring/input device 200 could be any computing component capable of communicating with or on a cellular network, an internet multimedia subsystem, and/or an IP network. One of skill in the art will recognize that the systems and methods described herein can also be used with a variety of electronic devices, such as, for example, tablet computers, desktops, servers, and other network connected devices.

The monitoring/input device 200 can comprise several components to execute various above-mentioned functions. The monitoring/input device 200 can comprise memory 802 including an operating system (OS) 804 and one or more standard applications 806. The standard applications 806 can include applications to control the various components of the monitoring/input device 200. In this case, the standard applications 806 can also comprise a monitoring application 830 and an imaging application 832. The monitoring application 830 may be instantiated to control the operation of the monitoring/input device 200 for detecting signals generated from a body. The control may also include the determination of which nodes receive what signals and the storage of that data. The imaging application 832 may be instantiated to configure the monitoring/input device 200 to act as an imaging device, whereby one or more of the nodes are energized to input electrical energy. For example, if instantiated, the imaging application 832 may cause the monitoring/input device 200 to applying a current to one or more of the nodes, record potentials of nodes not receiving a current, and constructing an image from the potentials.

In this method, the monitoring/input device 200 or the monitoring service 502 (or another device) defines a subset of nodes 102 to which a current is applied. Then monitoring/input device 200 records potentials on the nodes 102 that do not receive the current. Optionally, several subsets of the nodes 102 having different patterns are defined successively and resulting potentials are recorded successively. In other words, sets of the nodes 102 are changed and the application of current is repeated with different nodes 102. The monitoring/input device 200 or the monitoring service 502 determines an image of the part 202 of the body of the subject, for instance with an image reconstruction algorithm. Such method is suitable for noninvasive imaging such as electrical impedance tomography (EIT), in absolute (a-EIT), time difference (td-EIT) or multifrequency (MF-EIT) mode. Various part of the body may be imaged with this method, in particular lung, muscles, breast, cervix, brain, bladder, or limb. This method may be used for imaging volume variation of body parts, in particular under blood flow or perfusion.

In another example, one or more of the pads 402 of the nodes 102 may be replaced by other types of electromagnetic energy emitters, such as an infrared, visible, or near infrared light emitting diode (LED). In some examples, as explained above, the nodes 102 may be emitters, sensors, or a coupling of an emitter/sensor. For example, a coupled emitter/sensor may be used to acquire a signal in the case of a non-self-emitted physiologic signal. The near infrared emitters can be used in processes such as near infrared spectroscopy or optical coherence tomography. The standard applications 806 can also include one or more functions or operations as those described in FIGS. 1-8, above. In some further examples, one or more of the nodes 102 may be ultrasonic transducers coupled to be used in applications such as echography applications. As used herein, an ultrasonic transducer may be a transmitter, receiver, and/or transceiver.

The monitoring/input device 200 may also comprise one or more processors 812 and one or more of removable storage 814, non-removable storage 816, transceiver(s) 818, output device(s) 820, and input device(s) 822. In various implementations, the memory 802 can be volatile (such as random-access memory (RAM)), non-volatile (such as read only memory (ROM), flash memory, etc.), or some combination of the two. The memory 802 may be used to store various data received from the electrode set 100 and/or data received from the monitoring service 502 through the network 504.

The memory 802 can also include the OS 804. The OS 804 contains the modules and software that support basic functions, such as scheduling tasks, executing applications, and controlling peripherals. In some examples, the OS 804 can enable the monitoring application 830, the imaging application 832, and provide other functions, as described above, via the transceiver(s) 818. The OS 804 can also enable the monitoring/input device 200 to send and retrieve other data and perform other functions. It should be noted that one or more functions of the presently disclosed subject matter may be executed by other systems than the OS 804, such as firmware/FPGA/ASIC.

The monitoring/input device 200 can also comprise one or more processors 812. In some implementations, the processor(s) 812 can be a central processing unit (CPU), a graphics processing unit (GPU), both CPU and GPU, or any other processing unit such as an application-specific integrated circuit (ASIC) or Field Programmable Gate Arrays (FPGA), by way of example and not by way of limitation. The monitoring/input device 200 may also include additional data storage devices (removable and/or non-removable) such as, for example, magnetic disks, optical disks, or tape. Such additional storage is illustrated in FIG. 8 by removable storage 814 and non-removable storage 816.

Non-transitory computer-readable media may include volatile and nonvolatile, removable and non-removable tangible, physical media implemented in technology for storage of information, such as computer readable instructions, data structures, program modules, or other data. The memory 802, removable storage 814, and non-removable storage 816 are all examples of non-transitory computer-readable media. Non-transitory computer-readable media include, but are not limited to, RAM, ROM, electronically erasable programmable ROM (EEPROM), flash memory or other memory technology, compact disc ROM (CD-ROM), digital versatile discs (DVD) or other optical storage, magnetic cassettes, magnetic tape, magnetic disk storage or other magnetic storage devices, or any other tangible, physical medium which can be used to store the desired information and which can be accessed by the monitoring/input device 200. Any such non-transitory computer-readable media may be part of the monitoring/input device 200 or may be a separate database, databank, remote server, or cloud-based server.

In some implementations, the transceiver(s) 818 include any transceivers known in the art. In some examples, the transceiver(s) 818 can include wireless modem(s) to facilitate wireless connectivity with other components (e.g., between the monitoring/input device 200 and the network 504), the Internet, and/or an intranet, as well as wireless network adapters or other capable equipment.

The transceiver(s) 818 may also include one or more radio transceivers that perform the function of transmitting and receiving radio frequency communications via an antenna (e.g., Wi-Fi or Bluetooth^{®}). In other examples, the transceiver(s) 818 may include wired communication components, such as a wired modem or Ethernet port, for communicating via one or more wired networks. The transceiver(s) 818 can enable the monitoring/input device 200 to download files, access web applications, and provide other communications associated with the systems and methods, described above.

In some implementations, the output device(s) 820 include any output devices known in the art, such as a display (e.g., a liquid crystal or thin-film transistor (TFT) display), a touchscreen, speakers, a vibrating mechanism, or a tactile feedback mechanism. Thus, the output device(s) can include a screen, or display. The output device(s) 820 can also include speakers, or similar devices, to play sounds or ringtones when an audio call or video call is received. Output device(s) 820 can also include ports for one or more peripheral devices, such as headphones, peripheral speakers, or a peripheral display.

In various implementations, input device(s) 822 include any input devices known in the art. For example, the input device(s) 822 may include one or more components of the electrode set 100. In another example, the input device(s) 822 may include a camera, a microphone, or a keyboard/keypad. The input device(s) 822 can include a touch-sensitive display or a keyboard to enable users to enter data and make requests and receive responses via web applications (e.g., in a web browser), make audio and video calls, and use the standard applications 806, among other things. For example, the monitoring/input device 200 may be a cellular telephone having input ports capable of receiving data from the electrode set 100. The touch-sensitive display or keyboard/keypad may be a standard push button alphanumeric multi-key keyboard (such as a conventional QWERTY keyboard), virtual controls on a touchscreen, or one or more other types of keys or buttons, and may also include a joystick, wheel, and/or designated navigation buttons, or the like.

The presently disclosed examples are considered in all respects to be illustrative and not restrictive. The scope of the invention is indicated by the appended claims, rather than the foregoing description.

## Claims

1. An electrode set (100), the electrode set (100) comprising:
a plurality of nodes (102) comprising a plurality of first nodes (102) and a plurality of second
nodes (102), each of the first nodes (102) comprising a first pad (402) for receiving electromagnetic energy from a first portion of a part being studied, each of the second nodes (102) comprising a second pad (402) for receiving electromagnetic energy from a second portion of the part being studied or for sending a signal into the first portion of the part being studied;
a plurality of connectors (104) each connecting one of the first nodes (102) to one of the second nodes (102);
a first wire (302) within the connector (104), the first wire (302) in electrical communication with the first pad (402) of the first node (102) and extending to a measurement lead (106); and
a second wire (302) within the connector (104), the second wire (302) in electrical communication with the second pad (402) of the second node (102) and extending to the measurement lead (106), wherein the measurement lead (106) terminates at a measurement connector (104) to be inserted into a monitoring/input device,
wherein said electrode set (100) further comprises a plurality of alignment markers (110) each affixed to one of the first nodes (102) and the second nodes (102), each of the alignment markers (110) being configured to be removably affixed to a landmark on the subject of the part being studied and to transfer a pull force in a direction of a force vector to either the first node (102) or the second node (102) as the alignment marker (110) is being affixed to the landmark, and
in that each of the connectors (104) is formed from a shape that provides for a consistent deformation from a first distance between the first node (102) and the second node (102) to one or more second distances between the first node (102) and the second node (102) as the pull force is applied to the first node (102) or the second node (102), the consistent deformation causing the first and second nodes (102) to space apart in the direction of the force vector to allow for a placement of the first and second nodes (102) on the locations of the body part to be measured, wherein in an undeformed state the first node (102) and the second node (102) are planar and in a deformed state the first node (102) and the second node (102) are non-planar, wherein a length of the connector (104) remains constant from the undeformed state to the deformed state.

2. The electrode set according to claim **1,** further comprising:
a plurality of third nodes (102), at least a portion of the plurality of third nodes (102) comprising a third pad (402) for receiving electromagnetic energy from a plurality of third portions of the part being studied; and
a plurality of second connectors (104) connecting at least one of the plurality of third nodes (102) to either the first node (102) or the second node (102), the plurality of second connectors (104) formed from the shape that provides for consistent deformation from a first distance between at least a portion of the plurality of third nodes (102) to one or more second distances between at least a portion of the plurality of third nodes (102) as a pull force is applied to the at least one alignment marker (110), wherein in an undeformed state the plurality of third nodes (102) are planar and in a deformed state the plurality of third nodes (102) are non-planar.

3. The electrode set according to claim **2,** further comprising a plurality of second alignment markers (110) affixed to one or more of the plurality of third nodes (102), the plurality of second alignment markers (110) to be removably affixed to a plurality of second landmarks on the subject of the part being studied, wherein the plurality of second alignment markers (110) are configured to transfer the pull force to at least a portion of the plurality of third nodes (102) as the plurality of second alignment markers (110) are being affixed to the landmark.

4. The electrode set according to any one of claims **1** to **3,** further comprising a first pad stiffener (406) affixed to the first node (102), wherein the first pad stiffener (406) secures an electrical connection between the first pad (402) and the first wire (302).

5. The electrode set according to any one of claims **1** to **4,** wherein the at least one connector (104) comprises a plastic substrate, polyimide, polyethylene, polyether ether ketone (PEEK), or a non-conductive polyester or polymer.

6. The electrode set according to any one of claims **1** to **5,** wherein the shape comprises a sinusoidal shape.

7. The electrode set according to any one of claims **1** to **6,** wherein the shape comprises a spiral, a double spiral, a horseshoe, or an angular shape.

8. The electrode set according to any one of claims **1** to **7,** wherein the pad (402) comprises conductive or semi-conductive materials.

9. The electrode set according to any one of claims **1** to **8,** wherein the first pad (402) and/or the second pad (402) comprises copper, aluminum, stainless steel, gold, silver, or alloys thereof.

10. The electrode set according to any one of claims **1** to **9,** wherein the first wire (302) and/or the second wire (302) comprise(s) copper, aluminum, stainless steel, gold, silver, or alloys thereof.

11. The electrode set according to any one of claims **1** to **10,** wherein the first pad (402) comprises a first passing hole (408) and the second pad (402) comprises a second passing hole (408), the first passing hole (408) and the second passing hole (408) being openings that allow for an injection of an electroconductive material enhancing electrical contact with skin.

12. The electrode set according to any one of claims **1** to **11,** wherein the electromagnetic energy is near infrared light, infrared light, or visible light.

13. The electrode set according to any one of claims **2** to **12,** further comprising a fourth node (102), wherein the fourth node (102) comprises a fourth pad (402) to receive electromagnetic energy resulting from the electromagnetic transmission from the third pad (402).

14. The electrode set according to any one of claims **2** to **13,** wherein at least one third node (102) is configured for receiving a sensor.

15. A method of measuring electrophysiological signals of a part of a subject, the method comprising:
placing an electrode set (100), according to any one of claims **1** to **14,** proximate to the part to be measured,
affixing the alignment markers (110) to landmarks on the subject of the part being studied, each of the alignment markers transferring a pull force in a direction of a force vector to either the first node (102) or the second node (102), each of the connectors (104) providing for a consistent deformation from a first distance between the first node (102) and the second node (102) to one or more second distances between the first node (102) and the second node (102), the first and second nodes (102) spacing apart in the direction of the force vector to allow for a placement of the first and second nodes (102) on the locations of the body part to be measured;
connecting measurement lead (106) to a monitoring/input device; and
instantiating a monitoring application in the monitoring/input device to commence measuring the electrophysiological signals of the part of the subject.

16. The method according to claim **15,** further comprising injecting an electroconductive, sonic, or light transmitting material through a first passing hole (408) of the first pad (402) and a second passing hole of the second pad (402).

17. A sensor set system (500) comprising:
- a sensor set (100) according to any one of claims **1** to **14,** wherein the monitoring/input device is configured to receive data of electrophysiological signals received from the first pad or the second pad; and
- a monitoring service in communication with the monitoring/input device for receiving the data of the electrophysiological signals or transmitting instructions to the monitoring/input device.

## Patentansprüche

1. Elektrodensatz (100), wobei der Elektrodensatz (100) umfasst:
eine Vielzahl von Knoten (102), die eine Vielzahl von ersten Knoten (102) und eine Vielzahl von zweiten Knoten (102) umfassen, wobei jeder der ersten Knoten (102) ein erstes Pad (402) zum Empfangen elektromagnetischer Energie von einem ersten Abschnitt eines untersuchten Teils umfasst, wobei jeder der zweiten Knoten (102) ein zweites Pad (402) zum Empfangen elektromagnetischer Energie von einem zweiten Abschnitt des untersuchten Teils oder zum Senden eines Signals in den ersten Abschnitt des untersuchten Teils umfasst;
eine Vielzahl von Verbindern (104), die jeweils einen der ersten Knoten (102) mit einem der zweiten Knoten (102) verbinden;
einen ersten Draht (302) innerhalb des Verbinders (104), wobei der erste Draht (302) in elektrischer Verbindung mit dem ersten Pad (402) des ersten Knotens (102) steht und sich zu einer Messleitung (106) erstreckt; und
einen zweiten Draht (302) innerhalb des Verbinders (104), wobei der zweite Draht (302) in elektrischer Verbindung mit dem zweiten Pad (402) des zweiten Knotens (102) steht und sich zur Messleitung (106) erstreckt, wobei die Messleitung (106) an einem Messverbinder (104) endet, der in eine Überwachungs-/Eingabevorrichtung einzuführen ist,
wobei der Elektrodensatz (100) weiter eine Vielzahl von Ausrichtungsmarkierungen (110) umfasst, die jeweils an einem der ersten Knoten (102) und der zweiten Knoten (102) fixiert sind, wobei jede der Ausrichtungsmarkierungen (110) konfiguriert ist, um abnehmbar an einem Orientierungspunkt am Subjekt des untersuchten Teils fixiert zu werden und eine Zugkraft in eine Richtung eines Kraftvektors an entweder den ersten Knoten (102) oder den zweiten Knoten (102) zu übertragen, wenn die Ausrichtungsmarkierung (110) an dem Orientierungspunkt fixiert ist, und
dadurch, dass jeder der Verbinder (104) aus einer Form gebildet ist, die eine einheitliche Verformung von einem ersten Abstand zwischen dem ersten Knoten (102) und dem zweiten Knoten (102) auf einen oder mehrere zweite Abstände zwischen dem ersten Knoten (102) und dem zweiten Knoten (102) bereitstellt, wenn die Zugkraft auf den ersten Knoten (102) oder den zweiten Knoten (102) angelegt wird, wobei die einheitliche Verformung den ersten und zweiten Knoten (102) veranlasst, sich in Richtung des Kraftvektors voneinander zu entfernen, um eine Platzierung des ersten und zweiten Knotens (102) an den Stellen des zu messenden Körperteils zu ermöglichen, wobei der erste Knoten (102) und der zweite Knoten (102) in einem unverformten Zustand planar sind, und der erste Knoten (102) und der zweite Knoten (102) in einem deformierten Zustand nicht planar sind, wobei eine Länge des Verbinders (104) vom unverformten Zustand in den verformten Zustand konstant bleibt.

2. Elektrodensatz nach Anspruch **1,** weiter umfassend:
eine Vielzahl von dritten Knoten (102), wobei mindestens ein Abschnitt der Vielzahl von dritten Knoten (102) ein drittes Pad (402) zum Empfangen elektromagnetischer Energie von einer Vielzahl von dritten Abschnitten des untersuchten Teils umfasst;
und
eine Vielzahl von zweiten Verbindern (104), die mindestens einen der Vielzahl von dritten Knoten (102) entweder mit dem ersten Knoten (102) oder dem zweiten Knoten (102) verbinden, wobei die Vielzahl von zweiten Verbindern (104) aus der Form gebildet sind, die eine einheitliche Verformung aus einem ersten Abstand zwischen mindestens einem Abschnitt der Vielzahl von dritten Knoten (102) in einen oder mehrere zweite Abstände zwischen mindestens einem Abschnitt der Vielzahl von dritten Knoten (102) bereitstellt, wenn eine Zugkraft auf die mindestens eine Ausrichtungsmarkierung (110) angelegt wird, wobei die Vielzahl von dritten Knoten (102) in einem unverformten Zustand planar sind und die Vielzahl von dritten Knoten (102) in einem verformten Zustand nicht planar sind.

3. Elektrodensatz nach Anspruch **2,** der weiter eine Vielzahl von zweiten Ausrichtungsmarkierungen (110) umfasst, die an einem oder mehreren der Vielzahl von dritten Knoten (102) fixiert sind, wobei die Vielzahl von zweiten Ausrichtungsmarkierungen (110) abnehmbar an einer Vielzahl von zweiten Orientierungspunkten an der Person des untersuchten Teils angebracht sind, wobei die Vielazahl von zweiten Ausrichtungsmarkierungen (110) konfiguriert sind, um die Zugkraft auf mindestens einen Abschnitt der Vielzahl von dritten Knoten (102) zu übertragen, wenn die Vielzahl von zweiten Ausrichtungsmarkierungen (110) am Orientierungspunkt fixiert sind.

4. Elektrodensatz nach einem der Ansprüche **1** bis **3,** der weiter eine erste Padversteifung (406) umfasst, die am ersten Knoten (102) fixiert ist, wobei die erste Padversteifung (406) eine elektrische Verbindung zwischen dem ersten Pad (402) und dem ersten Draht (302) sichert.

5. Elektrodensatz nach einem der Ansprüche **1** bis **4,** wobei der mindestens eine Verbinder (104) ein Kunststoffsubstrat, Polyimid, Polyethylen, Polyetheretherketon (PEEK) oder ein nicht leitfähiges Polyester oder Polymer umfasst.

6. Elektrodensatz nach einem der Ansprüche **1** bis **5,** wobei die Form eine Sinusform umfasst.

7. Elektrodensatz nach einem der Ansprüche **1** bis **6,** wobei die Form eine Spirale, eine Doppelspirale, ein Hufeisen oder eine Winkelform umfasst.

8. Elektrodensatz nach einem der Ansprüche **1** bis **7,** wobei das Pad (402) leitende oder halbleitende Materialien umfasst.

9. Elektrodensatz nach einem der Ansprüche **1** bis **8,** wobei das erste Pad (402) und/oder das zweite Pad (402) Kupfer, Aluminium, Edelstahl, Gold, Silber oder Legierungen davon umfasst.

10. Elektrodensatz nach einem der Ansprüche **1** bis **9,** wobei der erste Draht (302) und/oder der zweite Draht (302) Kupfer, Aluminium, Edelstahl, Gold, Silber oder Legierungen davon umfasst.

11. Elektrodensatz nach einem der Ansprüche **1** bis **10,** wobei das erste Pad (402) ein erstes Durchgangsloch (408) umfasst und das zweite Pad (402) ein zweites Durchgangsloch (408) umfasst, wobei das erste Durchgangsloch (408) und das zweite Durchgangsloch (408) Öffnungen sind, die eine Injektion eines elektrisch leitenden Materials zur Verbesserung des elektrischen Kontakts mit der Haut ermöglichen.

12. Elektrodensatz nach einem der Ansprüche **1** bis **11,** wobei die elektromagnetische Energie Nahinfrarotlicht, Infrarotlicht oder sichtbares Licht ist.

13. Elektrodensatz nach einem der Ansprüche **2** bis **12,** der weiter einen vierten Knoten (102) umfasst, wobei der vierte Knoten (102) ein viertes Pad (402) umfasst, um elektromagnetische Energie zu empfangen, die sich aus der elektromagnetischen Übertragung vom dritten Pad (402) ergibt.

14. Elektrodensatz nach einem der Ansprüche **2** bis **13,** wobei mindestens ein dritter Knoten (102) zum Empfangen eines Sensors konfiguriert ist.

15. Verfahren zum Messen elektrophysiologischer Signale eines Teils eines Subjekts, wobei das Verfahren umfasst:
Platzieren eines Elektrodensatzes (100) nach einem der Ansprüche **1** bis **14** in der Nähe des zu messenden Teils,
Fixieren der Ausrichtungsmarkierungen (110) an Orientierungspunkten am Subjekt des untersuchten Teils, wobei jede der Ausrichtungsmarkierungen eine Zugkraft in eine Richtung eines Kraftvektors entweder an den ersten Knoten (102) oder den zweiten Knoten (102) überträgt, wobei jeder der Verbinder (104) eine einheitliche Verformung von einem ersten Abstand zwischen dem ersten Knoten (102) und dem zweiten Knoten (102) auf einen oder mehrere zweite Abstände zwischen dem ersten Knoten (102) und dem zweiten Knoten (102) bereitstellt, wobei sich der erste und zweite Knoten (102) in der Richtung des Kraftvektors voneinander entfernen, um eine Platzierung des ersten und zweiten Knotens (102) an den Stellen des zu messenden Körperteils zu ermöglichen;
Verbinden der Messleitung (106) mit einer Überwachungs-/Eingabevorrichtung; und
Instanziieren einer Überwachungsanwendung in der Überwachungs-/Eingabevorrichtung, um mit der Messung der elektrophysiologischen Signale des Teils des Subjekts zu beginnen.

16. Verfahren nach Anspruch **15,** das weiter Injizieren eines elektroleitenden, schall- oder lichtdurchlässigen Materials durch ein erstes Durchgangsloch (408) des ersten Pads (402) und ein zweites Durchgangsloch des zweiten Pads (402) umfasst.

17. Sensorsatzsystem (500), umfassend:
- einen Sensorsatz (100) nach einem der Ansprüche **1** bis **14,** wobei die Überwachungs-/Eingabevorrichtung konfiguriert ist, um Daten von elektrophysiologischen Signalen zu empfangen, die vom ersten Pad oder dem zweiten Pad empfangen werden; und
- einen Überwachungsdienst in Verbindung mit der Überwachungs-/Eingabevorrichtung zum Empfangen der Daten der elektrophysiologischen Signale oder Senden von Anweisungen an die Überwachungs-/Eingabevorrichtung.

## Revendications

1. Ensemble d'électrodes (100), l'ensemble d'électrodes (100) comprenant :
une pluralité de nœuds (102) comprenant une pluralité de premiers nœuds (102) et une pluralité de deuxièmes nœuds (102), chacun des premiers nœuds (102) comprenant un premier tampon (402) pour recevoir de l'énergie électromagnétique en provenance d'une première portion d'une partie qui est étudiée, chacun des deuxièmes nœuds (102) comprenant un deuxième tampon (402) pour recevoir de l'énergie électromagnétique en provenance d'une deuxième portion de la partie qui est étudiée ou pour envoyer un signal dans la première portion de la partie qui est étudiée ;
une pluralité de connecteurs (104) connectant chacun un des premiers nœuds (102) à un des deuxièmes nœuds (102) ;
un premier câble (302) à l'intérieur du connecteur (104), le premier câble (302) étant en communication électrique avec le premier tampon (402) du premier nœud (102) et s'étendant jusqu'à un fil de mesure (106) ; et
un second câble (302) à l'intérieur du connecteur (104), le second câble (302) étant en communication électrique avec le deuxième tampon (402) du deuxième nœud (102) et s'étendant jusqu'au fil de mesure (106), dans lequel le fil de mesure (106) se termine au niveau d'un connecteur (104) de mesure à insérer dans un dispositif de surveillance/d'entrée,
dans lequel ledit ensemble d'électrodes (100) comprend en outre une pluralité de repères d'alignement (110) fixés chacun à un des premiers nœuds (102) et des deuxièmes nœuds (102), chacun des repères d'alignement (110) étant configuré pour être fixé de manière amovible à un point de repère sur le sujet de la partie qui est étudiée et pour transférer une force de traction dans une direction d'un vecteur de force soit au premier nœud (102), soit au deuxième nœud (102), alors que le repère d'alignement (110) est fixé au point de repère, et
en ce que chacun des connecteurs (104) est constitué d'une forme qui fournit une déformation régulière depuis une première distance entre le premier nœud (102) et le deuxième nœud (102) jusqu'à une ou plusieurs secondes distances entre le premier nœud (102) et le deuxième nœud (102) alors que la force de traction est appliquée sur le premier nœud (102) ou le deuxième nœud (102), la déformation régulière amenant les premier et deuxième nœuds (102) à être espacés dans la direction du vecteur de force pour permettre un positionnement des premier et deuxième nœuds (102) sur les emplacements de la partie de corps à mesurer, dans lequel, dans un état non déformé, le premier nœud (102) et le deuxième nœud (102) sont plats, et, dans un état déformé, le premier nœud (102) et le deuxième nœud (102) ne sont pas plats, dans lequel une longueur du connecteur (104) reste constante depuis l'état non déformé jusqu'à l'état déformé.

2. Ensemble d'électrodes selon la revendication **1,** comprenant en outre :
une pluralité de troisièmes nœuds (102), au moins une portion de la pluralité de troisièmes nœuds (102) comprenant un troisième tampon (402) pour recevoir de l'énergie électromagnétique en provenance d'une pluralité de troisièmes portions de la partie qui est étudiée ; et
une pluralité de seconds connecteurs (104) connectant au moins un de la pluralité de troisièmes nœuds (102) soit au premier nœud (102), soit au deuxième nœud (102), la pluralité de seconds connecteurs (104) étant constitués de la forme qui fournit une déformation régulière depuis une première distance entre au moins une portion de la pluralité de troisièmes nœuds (102) jusqu'à une ou plusieurs secondes distances entre au moins une portion de la pluralité de troisièmes nœuds (102) alors qu'une force de traction est appliquée sur l'au moins un repère d'alignement (110), dans lequel, dans un état non déformé, la pluralité de troisièmes nœuds (102) sont plats, et, dans un état déformé, la pluralité de troisièmes nœuds (102) ne sont pas plats.

3. Ensemble d'électrodes selon la revendication **2,** comprenant en outre une pluralité de seconds repères d'alignement (110) fixés à un ou plusieurs de la pluralité de troisièmes nœuds (102), la pluralité de seconds repères d'alignement (110) devant être fixés de manière amovible à une pluralité de seconds points de repère sur le sujet de la partie qui est étudiée, dans lequel la pluralité de seconds repères d'alignement (110) sont configurés pour transférer la force de traction à au moins une portion de la pluralité de troisièmes nœuds (102) alors que la pluralité de seconds repères d'alignement (110) sont fixés au point de repère.

4. Ensemble d'électrodes selon l'une quelconque des revendications **1** à **3,** comprenant en outre un premier raidisseur de tampon (406) fixé au premier nœud (102), dans lequel le premier raidisseur de tampon (406) assure une connexion électrique entre le premier tampon (402) et le premier câble (302).

5. Ensemble d'électrodes selon l'une quelconque des revendications **1** à **4,** dans lequel l'au moins un connecteur (104) comprend un substrat en plastique, du polyimide, du polyéthylène, du polyétheréthercétone (PEEK), ou un polyester ou un polymère non conducteurs.

6. Ensemble d'électrodes selon l'une quelconque des revendications **1** à **5,** dans lequel la forme comprend une forme sinusoïdale.

7. Ensemble d'électrodes selon l'une quelconque des revendications **1** à **6,** dans lequel la forme comprend une spirale, une double spirale, un fer à cheval, ou une forme angulaire.

8. Ensemble d'électrodes selon l'une quelconque des revendications **1** à **7,** dans lequel le tampon (402) comprend des matériaux conducteurs ou semi-conducteurs.

9. Ensemble d'électrodes selon l'une quelconque des revendications **1** à **8,** dans lequel le premier tampon (402) et/ou le deuxième tampon (402) comprennent du cuivre, de l'aluminium, de l'acier inoxydable, de l'or, de l'argent, ou des alliages de ceux-ci.

10. Ensemble d'électrodes selon l'une quelconque des revendications **1** à **9,** dans lequel le premier câble (302) et/ou le second câble (302) comprennent du cuivre, de l'aluminium, de l'acier inoxydable, de l'or, de l'argent, ou des alliages de ceux-ci.

11. Ensemble d'électrodes selon l'une quelconque des revendications **1** à **10,** dans lequel le premier tampon (402) comprend un premier trou de passage (408) et le deuxième tampon (402) comprend un second trou de passage (408), le premier trou de passage (408) et le second trou de passage (408) étant des ouvertures qui permettent une injection d'un matériau électroconducteur améliorant le contact électrique avec la peau.

12. Ensemble d'électrodes selon l'une quelconque des revendications **1** à **11,** dans lequel l'énergie électromagnétique est de la lumière infrarouge proche, de la lumière infrarouge, ou de la lumière visible.

13. Ensemble d'électrodes selon l'une quelconque des revendications **2** à **12,** comprenant en outre un quatrième nœud (102), dans lequel le quatrième nœud (102) comprend un quatrième tampon (402) pour recevoir de l'énergie électromagnétique résultant de la transmission électromagnétique depuis le troisième tampon (402).

14. Ensemble d'électrodes selon l'une quelconque des revendications **2** à **13,** dans lequel au moins un troisième nœud (102) est configuré pour recevoir un capteur.

15. Procédé de mesure de signaux électrophysiologiques d'une partie d'un sujet, le procédé comprenant :
le positionnement d'un ensemble d'électrodes (100), selon l'une quelconque des revendications **1** à **14,** à proximité de la partie à mesurer,
la fixation des repères d'alignement (110) à des points de repère sur le sujet de la partie qui est étudiée, chacun des repères d'alignement transférant une force de traction dans une direction d'un vecteur de force soit au premier nœud (102), soit au deuxième nœud (102), chacun des connecteurs (104) fournissant une déformation régulière depuis une première distance entre le premier nœud (102) et le deuxième nœud (102) jusqu'à une ou plusieurs secondes distances entre le premier nœud (102) et le deuxième nœud (102), les premier et deuxième nœuds (102) étant espacés dans la direction du vecteur de force pour permettre un positionnement des premier et deuxième nœuds (102) sur les emplacements de la partie de corps à mesurer ;
la connexion d'un fil de mesure (106) à un dispositif de surveillance/d'entrée ; et
l'instanciation d'une application de surveillance dans le dispositif de surveillance/d'entrée pour commencer à mesurer les signaux électrophysiologiques de la partie du sujet.

16. Procédé selon la revendication **15,** comprenant en outre l'injection d'un matériau électroconducteur, sonique ou transmettant la lumière à travers un premier trou de passage (408) du premier tampon (402) et un second trou de passage du deuxième tampon (402).

17. Système d'ensemble de capteurs (500) comprenant :
- un ensemble de capteurs (100) selon l'une quelconque des revendications **1** à **14,** dans lequel le dispositif de surveillance/d'entrée est configuré pour recevoir des données de signaux électrophysiologiques reçus en provenance du premier tampon ou du deuxième tampon ; et
- un service de surveillance en communication avec le dispositif de surveillance/d'entrée pour recevoir les données des signaux électrophysiologiques ou transmettre des instructions au dispositif de surveillance/d'entrée.
